Europäisches Patentamt

European Patent Office

Office européen des brevets

(11) Publication number: 0 301 528
A1

## (12) EUROPEAN PATENT APPLICATION

(21) Application number: 88112177.6

(22) Date of filing: 28.07.88

(51) Int. Cl.⁴: G01R 31/02

(30) Priority: 29.07.87 IT 4825187

(43) Date of publication of application:
01.02.89 Bulletin 89/05

(84) Designated Contracting States:
AT BE CH DE ES FR GB GR LI LU NL SE

(71) Applicant: VITROSELENIA S.P.A.
Via Tiburtina 1020
Rome(IT)

(72) Inventor: Cappellini, Luigi
Via Francesco Maria Greco 29
I-00168 Roma(IT)
Inventor: Cappellini, Daniele
V. le Adriatico, 50
I-00141 Roma(IT)
Inventor: Moretti, Cesarino
Via Alamanno Morelli, 10
I-00197 Roma(IT)

(74) Representative: Gustorf, Gerhard, Dipl.-Ing.
Patentanwalt Dipl.-Ing. Gerhard Gustorf
Mühlenstrasse 1
D-8300 Landshut(DE)

(54) Monitoring and warning system for series fed runway visual aids.

(57) System for failure detection of series fed lighting circuits which monitors the status of series fed lamps, providing for feed through short circuit and protection of the isolating transformer and suppression of overvoltage which appears at the failed lamp terminals.

Current monitoring systems become obsolete with the introduction of this invention, which is known as RLW (Runway Lighting Warning).

The RLW System, which consists basically of a control unit equipped with detector probes, utilizes the same series fed lamp supply line, without any need for supplementary lines, and displays the failed lamp identification number accurately. The system has its preferred application in series fed airport lighting systems, but may also find application within any series fed lighting system such as that of harbours, road networks and so an.

fig. 2

## Monitoring and warning system for series fed runway visual aids

The invention presented regards a system which monitors the lighting of an airport, or any other site which requires lighting, such as harbours, road networks, sporting grounds or others.

The invention finds its preferred application in the airport field bacause of the requirements for maximum safety which are correctly applied to this area.

It in fact solves the problem of centralized lamps serviceability check for runways and access paths.

This monitoring system is essentially an electronic facility which, without any additional cabling nor modifications thereof, makes use of the series connected lamps supply line and can display with accuracy the identification number of the failed lamp and provide for by-pass of the failed lamp and of the relevant isolating transformer and also for overvoltages snubbing.

The problem which this invention solves is mainly that of checking remotely all lamps fitted to the circuit and to display the failed lamp location number accurately, without making recourse to scheduled inspection routines.

In the event of one or more lamps failing, the system has a self-resetting device which short circuits the relevant isolating transformer and suppresses overvoltages, so as to eliminate the need for the replacement of fused film cutouts, which would otherwise burn out upon intervention.

The system therefore is essentially made up of a control unit and of one or more by pass and suppressor detectors, used separately or jointly in the same container and, when referred to previous solutions, provides safety and cost advantages.

In fact, previous solutions, may be summarized in:

1) systems which provide only indication of the percentage of failed lamps and therefore require inspection to locate single failed lamps, and therefore inspection routines, with consequential high costs;

2) systems which check each single lamp by means of detectors, interconnected to a data collection centre by means of additional lines, which results in higher costs and one at times impossible to implement.

The uniqueness of this invention is in the fact that the detector probes have built-in short circuit bypass and snubbers.

As a result, also effective protection to maintenance personnel is provided, even when the circuit is live.

The invention will now be described with reference to its presently preferred implementations, reported as a non limiting example and with reference to table drawings attached.

It is to be understood that the description to follow, which regards build and operation of the system, refers to the version preferred by the inventors; however there is nothing to prevent that the system is complemented in different manners, all based on the same philosophy of this invention. The invention will now be described with reference to the enclosed tables of drawings as non limiting example.

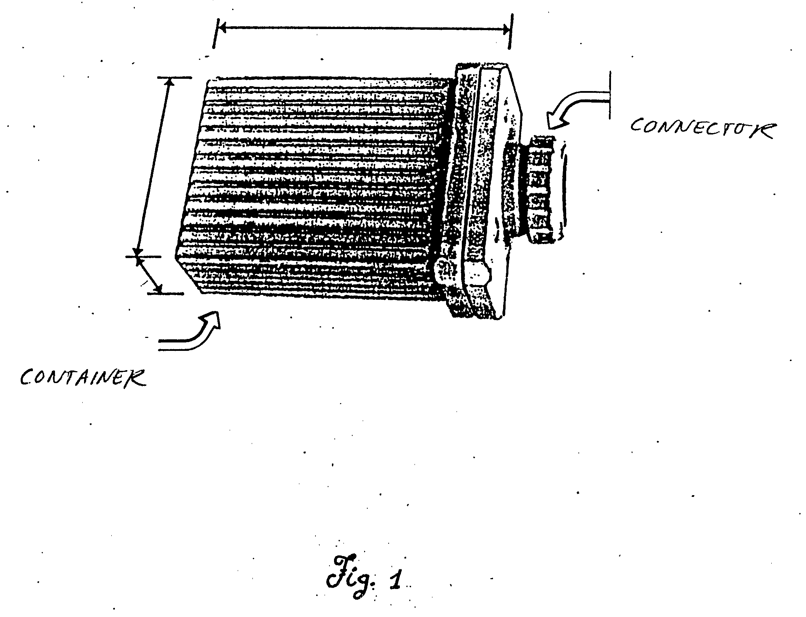

Figure 1 shows the architecture of the detector probe container made of insulating material, hermetically sealed, ribbed for thermal dissipation, containing the detector unit, the short circuit unit, the suppressor unit and a connector forming integral part of the container, (shown by the arrow on the right side of the container).

Figure 2 shows how detectors 1,2,3,4 are used with the control unit RLW 5, whereby sensors and unit are interconnected by a lighting circuit consisting of: a constant current power supply-regulator 6; series connected distribution system 7; pick-up 8.

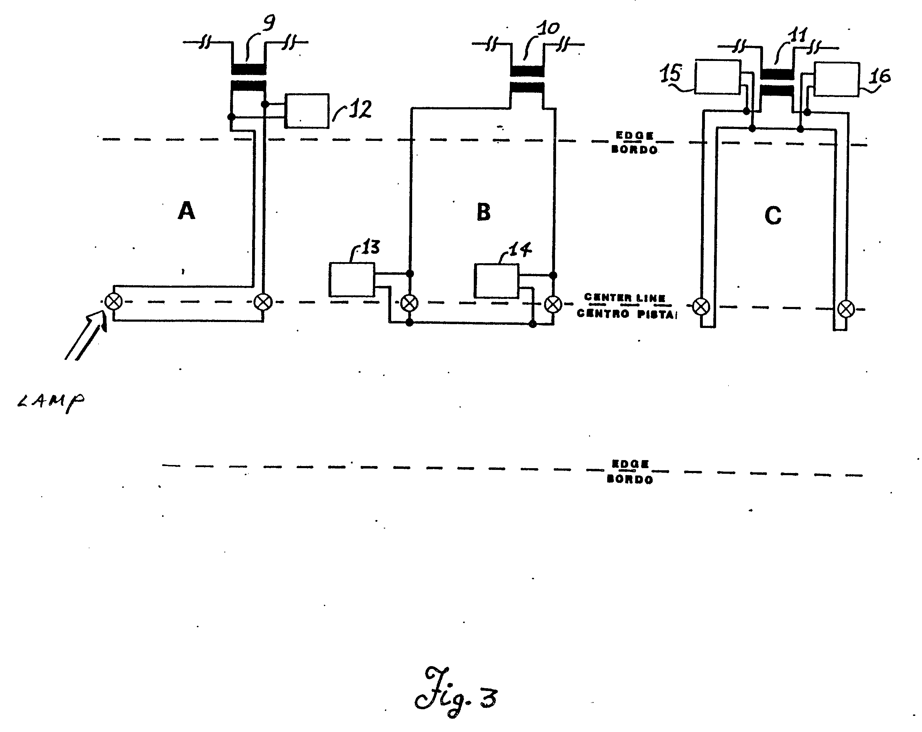

Figure 3 shows three typical schematics A.B,C for lamps fitted to the runway centre-line, i.e. far from the isolating transformers 9; 10, 11 and adopting relevant detectors 12,13,14,15,16.

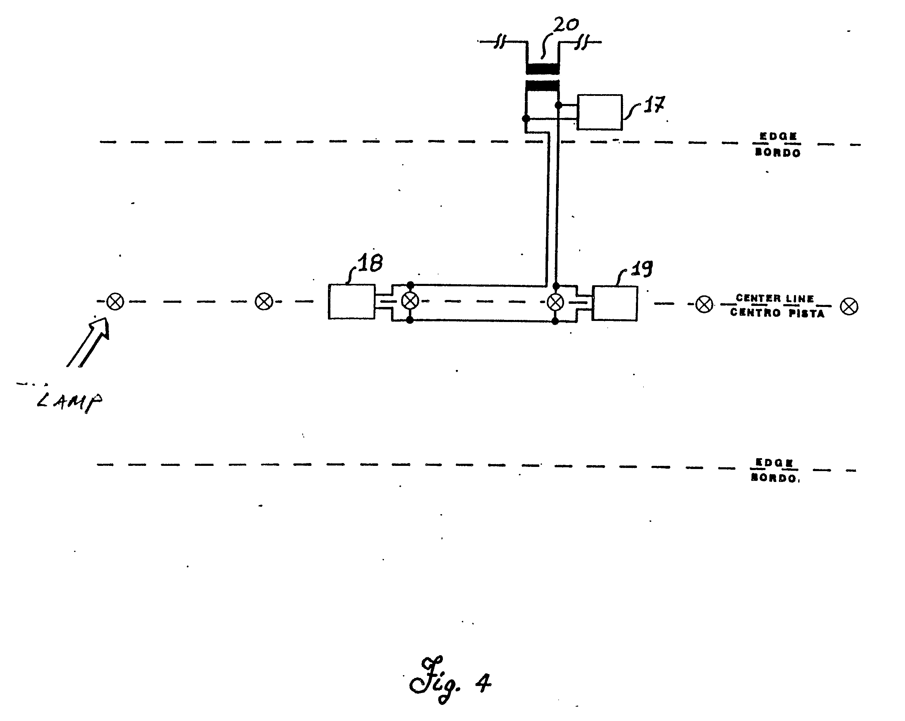

Figure 4 shows the schematics of detector 17 and of two by-pass and suppressor accessories 18,19 for lamps fitted to the runway centreline, i.e. far from isolating transformer 20.

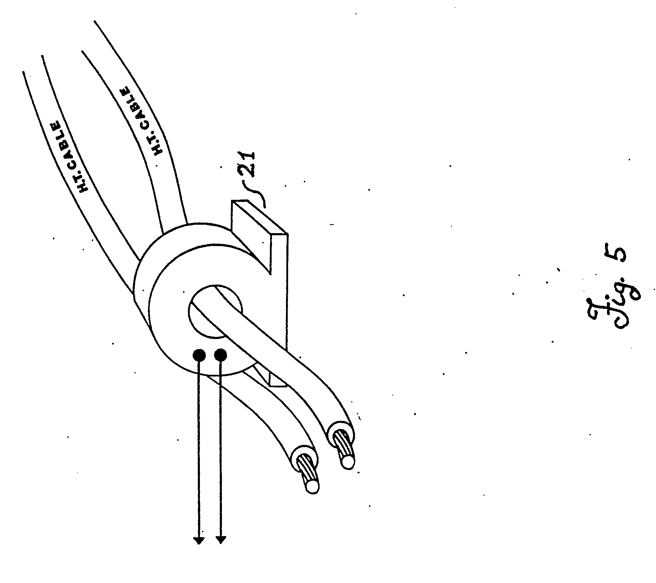

Figure 5 shows how pick-up 21 is coupled, without direct contact, with one of the high voltage lines.

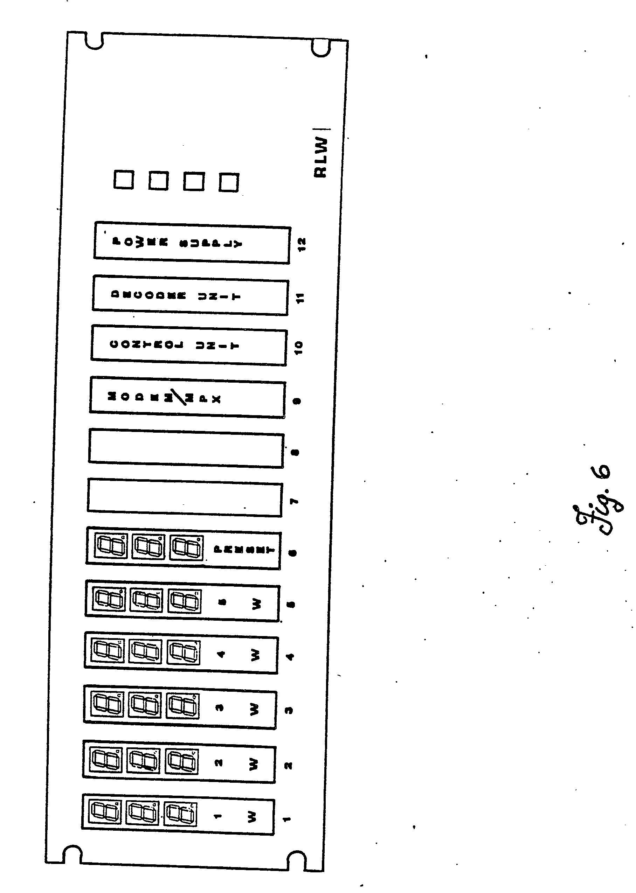

Figure 6 shows the RLW rack panel, which monitors a single constant current regulator.

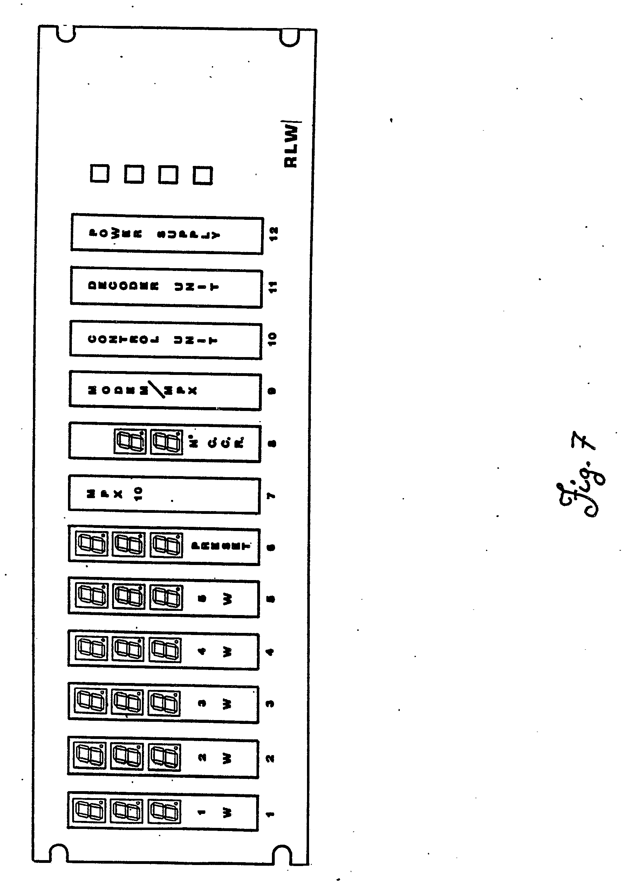

Figure 7 shows the RLW control panel configuration to monitor a number of constant current regulators.

Figure 8 shows the block diagram of the main units of the RLW control unit assembled to locate and warn of lamp serviceability status.
Here the following are visible:

22 Constant current regulator (dashed);
23 pick-up;
24 coupling circuits;
25,26 amplifiers;
27,30,32 comparators;
28 active integrator;
29 tuned circuit;
31 integrator;
33,40,41,43 AND circuits;
34 pulse detector;
35,36,37,38 counters;

39 programmable selectors;

42 resetting detector;

44 memory circuit;

45 decoupling circuit;

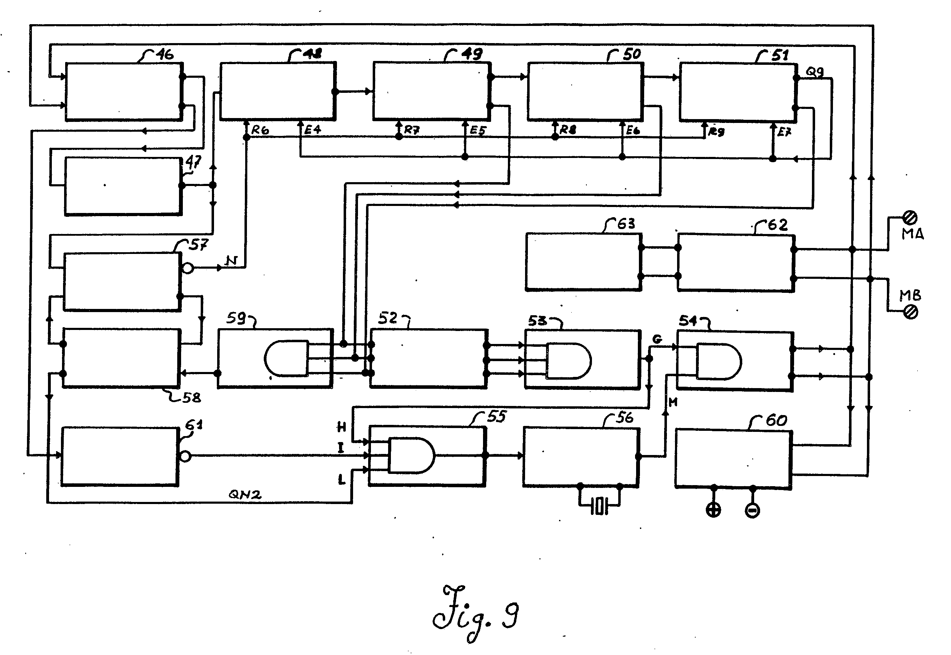

Figure 9 shows the block diagram of the main parts of the detectors which deliver the operating lamp signal to the control unit.

Here the following are visible:

46 coupling circuit;

47 pulse detector;

48,49,50,51 counters;

52 programmable selectors;

53,55,59 AND circuits;

54 transmitter;

56 quartz oscillator;

57 resetting detector;

58 memory circuit;

61 overvoltage sensor;

60 power supply;

62 first overvoltage suppressor;

63 second overvoltage suppressor and by-pass short circuit.

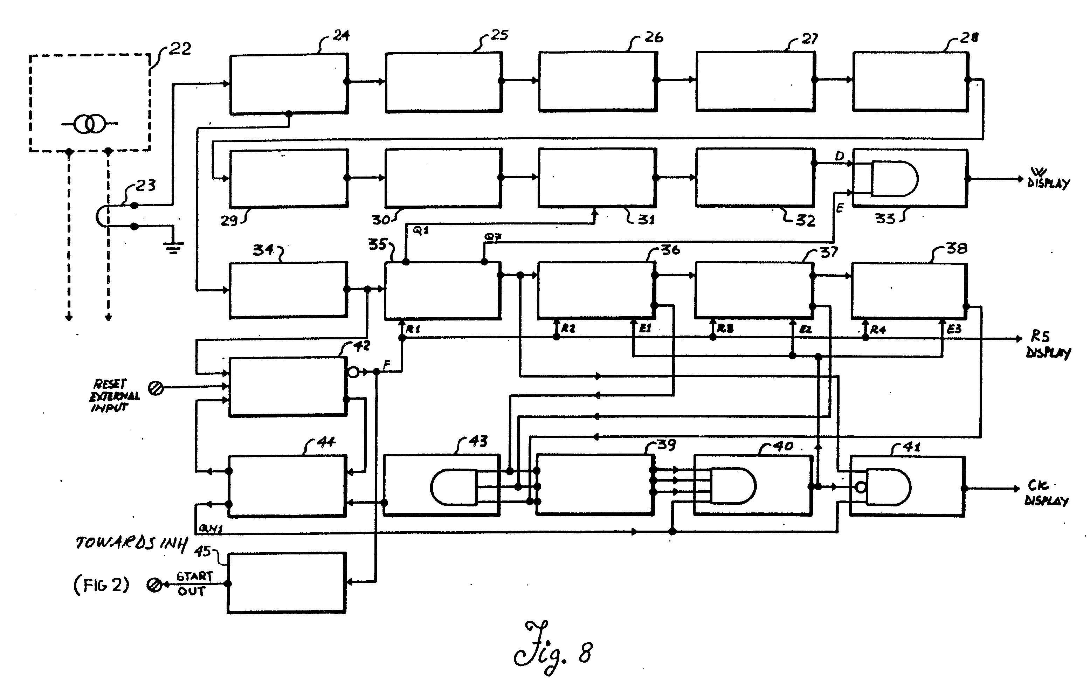

In the following, as a non limiting example, the description of the invention with reference to Figures 8 and 9, is given. Constant current regulator, 22 (Fig. 8) which forms part of the network surveilled, is shown dashed.

The system uses a reading head 23 which, inductively coupled to one of the high voltage cables, reads the lamps supply waveform and the envelope of the reply signal which all detectors in the system feed back to the cable, according to a pre-established sequence, only when the lamp is operational. If the lamp is burnt out or off for other reasons, the detector will not operate and therefore as the presence signal is missing, the control unit will provide a warning, as clarified in the following.

Information from pick-up 23 is coupled by a passive circuit with an impedance matching transformer 24 which has suitable suppressor divices which reduce amplitudes to prevent distruction of the other electronic circuits of the system. The first amplifier (25) has the task to preamplify, linearly, the weak reply signal which each detector in turn inserts on line and to present it with the correct amplitude at the second amplifier (26) input, which is a non linear, small signal amplifier and which passes information to comparator (27) only if its level is above typical noise floor present on the high voltage line.

Comparator 27 outputs the high frequency signals of the detectors together with the low frequency transients caused by closing and opening of the SCR and opening of the SCR semiconductors or of other components used in the constant current generator.

Therefore all signals, useful or not, are present at the output of comparator 27 only if their level is such to match the comparison voltage.

The purpose of comparator 27 is to ensure that the control unit is insensitive to low signals.

Eventually, comparator 27 provides squared signals, with constant amplitude, only if they pass a well defined value at the input.

All waveforms provided by comparator 27, either rectangular or square, are sent to circuit 28 which is an active integrator with suitable amplitude limiting feedback, which has the task to make their output triangular, at constant amplitude.

In particular, circuit 28 has the task to convert to triangular all square waves so that their lowest harmonic content lets tuned circuit 29 draw advantage of discerning, in terms of amplitude, at constant amplitudes appered, the frequency at which it resonates among lower, even or add harmonics.

Therefore, at the output of tuned circuit 29 there is only the resonating frequency which will appear with its highest sinus value and it alone will be comparable with and will emerge over, the comparison voltage of comparator 30.

Comparator 30 will provide a square wave at the resonating frequency which is the same as that used as detector reply and is sent to rectifier bridge 31 with a charge capacitor used as integrator, with a staircase charging pattern.

As each detector message includes a high frequency signal enveloped over a few mains cycles, the integrator charge time in circuit 31 is tailored so that only when 50% of mains cycles are passed, the integrator can reach the level required to regard the last comparator of chain 32, which will provide AND 33 with an enable signal for the entire duration of the detector signal, which is sent to input D and monitored at input E.

Coupling circuit 24 sends mains data to pulse detector 34 which has the task to generate a pulse for each mains cycle to drive first scaler 35, the output of which, suitably divided, provides the clock pulse to drive all following scalers 26,37, 38 and the timing for detectors to provide reply signals.

This first scaler 35 will also provide ancillary data such as: Q1 sent to integrator 31 which serves to reset the staircase integrator at the start of transmission cycle of each detector; Q7 sent to input E of AND 33 circuit, which serves to monitor, for short times, 70% of transmission time, whether output of comparator 32 is high or low so that the output from AND 33 circuit provides a warning signal W if D is low.

The control unit has the following scalers; 36 (units); 37 (tens) 38 (hundreds) which have the task to count the pulses provided by first counter 35. Three programming selectors 39, for units, tens and hundreds are programmed purposely to stop counting by circuit AND 40 when it has reached the number of detectors to be checked. Equally, by AND 41 circuit, also counting pulses CK and display drivers for warning indication which shift with counters 36,37,38 are stopped.

Pulses provided by detector 34 are integrated in circuit 42 and normally keep low output F of the reset detector circuit 42, so that counters 35,36,37,38 are stopped.

Pulses provided by detector 34 are integrated in circuit 42 and normally keep low output F of the reset detector circuit 42, so that counters 35,36,37,38 may operate.

Therefore circuit 42 has an integrator with a constant discharge time which, when supplies fail for one or more cycles, is such that circuit 42 resets R1, R2 R3, R4, R5 of all counting and measurement, including displays. This temporary lack of supplies may be purposely caused from outside when one wishes to update the monitoring by means of the Reset External input command.

Upon siwtch-on of the constant current generator reset detector circuit 42 also provides a soft start pulse to memory 44 so that QN1 inhibits output of driver CK of AND 41 toward displays.

This soft start device has been purposely fitted to give the fielded detector probes time enough to reach the required supply voltage and to perform monitoring at least 30″ from switch-on, which is considered the most critical for lamp filaments.

Monitoring will be performed when AND 43 circuit is enabled and that is only when their respective inputs are simultaneously high and coincident as a result of the delay set.

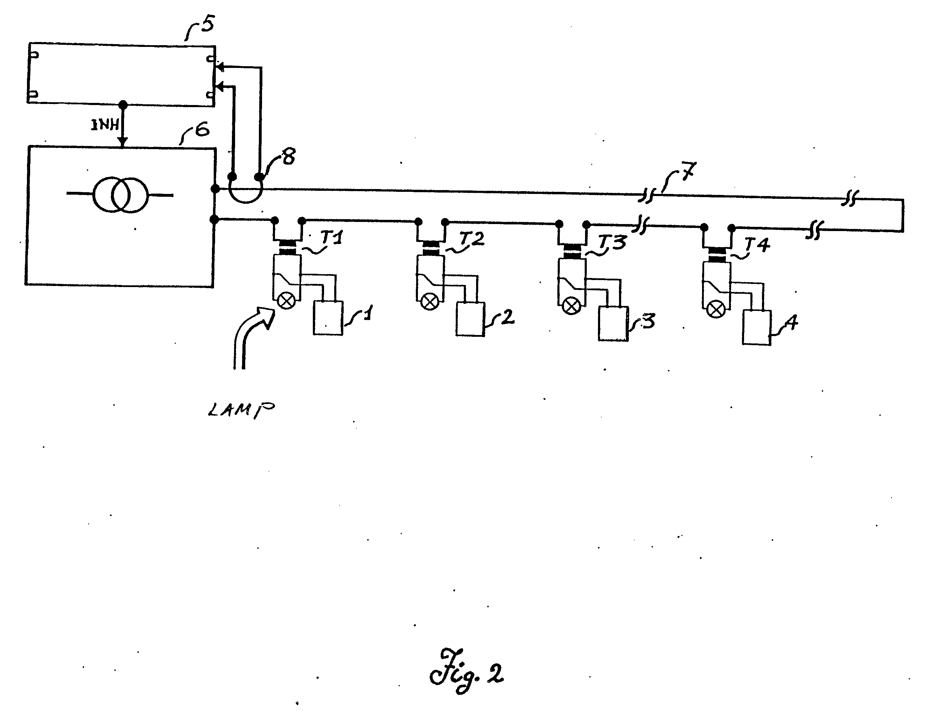

A suitable de-coupling circuit 45, provides a start out exit to be sent to inhibit input INH (Fig. 2) of the constant current generator.

Figure 9 shows in block form, the main functions accomplished, which are performed by the detector probes and which are needed to inspect, on-line, the signal which informs the control unit whether the lamp is serviceable. This figure also shows the by-pass and suppressor circuits.

It is clearly shown that the detector probe is wired in parallel with the lamp by means of clamps MA, MB (shown on the right side of the block diagram).

The reset circuit and its related counting, are almost identical and therefore synchronous with those housed within the control unit so that the detector probe, when reaching its enabling turn at the time set by the counter, can inject its signal on-line, so that the control unit will classify such time with an identification number by virtue of the fact that it adopts the same counter and moves in step with it.

The series (or non) waves which power the lamps, reagard a passive coupler 46, whose output drives pulse detector 47, which can extract, under all power conditions, a pulse for each mains cycle and used it to drive first prescalar 48, the output of which, suitably divided, will provide the clock pulse for all following scalers 49,50,51 and the timing for the detector probe to send the lamp status signal.

The probe is therefore provided with scalers 49 for units, 50 for tens, 51 for hundreds, which serve to count the pulses provided by prescaler 48. Three programming selector 52 for units, tens, hundreds are suitably set to form the correct identification number.

When the programmed number is reached, the three AND 53 inputs become high simultaneously, and only then AND 53 activates input G of transmitter 54 and input H of AND 55, which when its other inputs I and L allow, will activate quartz oscillator 56, which modulates input M of transmitter 54 for the entire period set by prescaler 48.

Scalers 48, 49, 50, 51 stop counting upon reaching full scale indication Q9 of counter 51 and by means of its inputs (enable) E4, E5, E6, E7 connected in parallel. This is for probes which do not have to operate cyclicly.

Counting and transmission may re-start at any time by effect of reset R6, R7, R8, R9 caused at each switch on of the constant current generator or when stimulated by the lack of one or more sinus supply cycles.

The mains pulses selected and present at the output of pulse detector 47 are integrated in circuit 57 and keep output N of reset detector 57 low so that scalers 48, 49, 50, 51 may operate.

The discharge constant of the integrator contained in 57 is equal to that of the control unit, and is such that in presence of a temporary power failure of more than one or more cycles, reset detector 57 resets counting, as also happens at the control unit.

Upon power up of the constant current generator feeding the lamps, reset detector 57 also provides for a soft start pulse to be sent to memory 58 so that QN2 inhibits input L of AND 55, so that quartz generator 56 is blocked and lamp serviceability signal is not transmitted.

As mentioned in the description of the control unit (Fig. 8) the soft start device has been purposely fitted to give supply 60 enough time to reach the required voltage and to monitor and transmit the signal after the 30″ delay set on the control unit.

Scaler resetting to start monitoring is given when allowed by AND 59, i.e. when its inputs are simultaneously high and coincident as a function of the delay set.

The overvoltage pulse detector 61 has the task to detect overvoltages due to failed lamp and to keep input I of AND 55 inhibited so that the probe cannot transmit, and such event is understood by the control unit as a warning of failed lamp. The detector probe has two accessory circuits 62 & 63 which have the function to snub the overvoltages present and to apply a short circuit between clamps MA and MB.

The first circuit 62 is essentially a snubber which captures the leading edge of the pulse, while the second parallel active circuit 63, with stands high transient currents and keeps clamps MA and MB shorted, replacing the failed lamp, so that current may circulate for long times or till normal conditions are restored, in which case the circuit is automatically reset.

**Claims**

1. Failure detection system for series fed lighting circuits, particularly suitable for airports, which is made up of a control unit (Figure 8), by one or more detector probes (Fig. 9), where the first (Fig. 8) is essentially formed by:
. constant current generator (dashed) 22;
. pick-up 23;
. coupling circuits 24;
. amplifiers 25, 26;
. comparators 27, 30, 32;
. active integrator 28;
. tuned circuit 29;
. integrator 31;
. AND circuits 33, 40, 41, 43;
. pulse detector 34;
. scalers 35, 36, 37, 38;
. programmable selectros 39;
. reset detector 42;
. memory circuit 44;
. decoupling current 45;
and the second, detector probes (Fig. 9) which include:
. coupling circuit 46;
. pulse detector 47;
. scalers 48, 49, 50, 51;
. programmable selectors 52;
. AND circuits 53, 55, 59;
. transmitter 54;
. quartz oscillator 56;
. reset detector 57;
. memory circuits 58;
. overvoltage detector 61;
. power supply 60;
. first snubber 62;
. a second snubber and by pass short circuit 63.

2. System for fault detection of series fed lighting circuits as per previous claim, where the detector probe, overvoltage suppressor, the by-pass short circuit device, the connector, may be housed in the same container.

3. System to detect failures in series fed illumination circuits, as per claim 2, where the detector probe container may preferably be made of policarbonate isolating material and/or similar, hermetically scaled and ribbed to provide for cooling, with a connector which is integral part of the same mould (Fig. 1).

4. System for the detection of failures on series fed lighting circuits, as per claims above, where probes 1, 2, 3 etc and control unit RLWS are interconnected by the same series fed lighting circuit, consisting of a constant current generator (6), of isolating transformers TI...TN, of a series distribution system 7 and of pick-up 8 (Figure 2).

5. System to detect faults on series fed lighting circuits as per previous claims, where the pick-up is coupled to the high voltage supply lines without any direct contact (Figure 5).

6. System to detect failures on a series fed lighting circuit as per previous claims, where the control unit RLW may manage one (Fig. 6) or more constant current generators (Fig. 7).

7. System for the detection of failures on series fed lighting circuits, as per previous claims, where the RLW may find application as well within airport facilities, also in harbour, road, railway networks.

8. System for fault detection of series fed lighting circuits, as per previous claims, where the system can carry out its supervising task by means of the lamp supply lines themselves, without any accessory line (Figure 2).

9. System for the detection of failures in series fed lighting circuits, as per previous claims, where the RLW system can update the lamp test by an inhibiting circuit INH (Fig. 2) which suppresses one or more supply cycles.

10. System for failure detection of series fed lighting circuits where the detector probes are connected directly to the terminals of the isolating transformer, without accessory lines to transmit data (lamp surveceability signal) (Figure 2, 3, 4).

11. System for failure detection of series fed lighting circuits, as per previous claims, where the RLW detector probes send signals only with serviceable lamp so that the control unit, only if the signal is missing, may provide failure indication, which may depend on:
- the lamp itself;
- on its isolating transformer;
- on its cables;
- on its lamp holder;
- on detector probe failure;
- on other causes.

12. Failure detector system for series fed lighting circuits, as per previous claims, where the detector probes of the RLW system deliver a signal only for serviceable lamp, applying a short circuit to the lamp terminals. at a frequency above that of the constant current generator.

13. Failure detector for series fed lighting circuits, as illustrated in the description above and in the drawings attached, as also any part thereof either detached or combined.

Fig. 1

INH

5

6

8

7

T1 T2 T3 T4

1 2 3 4

LAMP

Fig. 2

EP 0 301 528 A1

**9** **10** **11**

**15** **16**

**12**

EDGE
BORDO

A B C

**13** **14**

CENTER LINE
CENTRO PISTA

LAMP

EDGE
BORDO

*Fig. 3*

EP 0 301 528 A1

EDGE
BORDO

*18*

*17*

*20*

*19*

LAMP

CENTER LINE
CENTRO PISTA

EDGE
BORDO.

*Fig. 4*

EP 0 301 528 A1

H.T. CABLE

H.T. CABLE

21

Fig. 5

EP 0 301 528 A1

Fig. 6

EP 0 301 528 A1

W 1 | W 2 | W 3 | W 4 | W 5 | PRESET 6 | MPX 10 7 | N° C.C.R. 8 | MODEM/MPX 9 | CONTROL UNIT 10 | DECODER UNIT 11 | POWER SUPPLY 12

RLW

*Fig. 7*

Fig. 8

W DISPLAY

R5 DISPLAY

CK DISPLAY

RESET EXTERNAL INPUT

TOWARDS INH

(FIG 2)

START OUT

Fig. 9

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X | DE-A-3 012 726 (ALBERTUS et al.) * Page 13, last paragraph - page 4, paragraph 2; claim * | 1,4,5,7 ,8,10, 11,13 | G 01 R 31/02 |
| X | GB-A-2 074 328 (TOKYO SHIBAURA) * Abstract; figures 4,8 * | 1,8 | |
| A | DE-A-3 102 267 (LICENTIA) * Abstract * | 1 | |
| A | US-A-4 019 128 (REES) | | |

TECHNICAL FIELDS SEARCHED (Int. Cl.4)

G 01 R

The present search report has been drawn up for all claims

| Place of search | Date of completion of the search | Examiner |
|---|---|---|
| THE HAGUE | 07-11-1988 | HOORNAERT W. |

EPO FORM 1503 03.82 (P0401)